# EUROPEAN PATENT SPECIFICATION

(11) **EP 3 664 880 B1**
(45) Date of publication and mention of the grant of the patent: **11.10.2023**
(21) Application number: 18844176.0
(22) Date of filing: 01.08.2018
(51) Int. Cl.: A61M 16/04, A61F 13/12, A61M 25/02, A61M 16/10

(54) **TRACHEOSTOMA DEVICE HOLDER**
TRACHEOSTOMAVORRICHTUNGSHALTER
PORTE-DISPOSITIF DE TRACHÉOSTOMIE

(30) Priority: 11.08.2017 SE 1750985
(43) Date of publication of application: 17.06.2020
(73) Proprietor: Coloplast A/S, 3050 Humlebaek (DK)
(72) Inventor: SELVÖ, Thomas, 242 35 Hörby (SE); SVANTESSON, Lars, 242 94 Hörby (SE); ROJAS GARCIA, Maria, 221 51 Malmö (SE)
(86) International application number: PCT/SE2018/050782
(87) International publication number: WO 2019/032015

(56) References cited:
- EP-A1- 1 513 575
- EP-A1- 3 061 484
- WO-A1-03/016018
- WO-A1-2017/135867
- WO-A1-2017/135867
- WO-A1-2018/009118
- DE-U1-202004 007 566
- DE-U1-202004 007 566
- JP-A- 2013 052 033
- US-A- 5 819 734
- US-A- 5 819 734
- US-A1- 2005 277 901
- US-A1- 2008 072 911
- US-A1- 2008 072 911
- US-A1- 2009 223 521
- US-A1- 2010 307 504
- US-A1- 2011 162 650
- US-A1- 2012 103 341
- US-A1- 2012 103 341
- US-A1- 2015 306 327
- US-A1- 2015 306 327

## Description

### Field of the Invention

This invention pertains in general to a tracheostoma device holder. More particularly, the present invention pertains to a tracheostoma device holder, comprising a plate for attachment over a tracheostoma via the proximal side of the plate, said plate being provided with a through hole having a tracheostoma device fitting extending distally from the plate circumferentially of said through hole.

### Background of the Invention

A tracheostomy is a surgical procedure in which an opening is formed through the anterior surface of the neck into the trachea. The opening is referred to as a tracheostoma. A tracheostomy tube can be provided to extend between the tracheostoma and the trachea. A tracheostomy is performed for example when there is a malfunction, such as a result from injury or disorder, in respect of the nervous system or the respiratory passages, which malfunction results in an incapacity to obtain enough air. An inferior lung capacity or need of respiratory treatment may also result in a tracheostomy.

A laryngectomy is a surgical procedure, used for example to treat a carcinoma, which involves removal of the larynx or voice box and the creation of a tracheostoma. A consequence of the procedure is that the trachea is no longer connected to the pharynx but is diverted to the tracheostoma. After this procedure, normal nasal function is not possible. In a subject whose breathing functions normally, the nose and the mucous membrane lining of the nasal cavity perform important functions in conditioning inhaled air. The convoluted passages and rich blood supply serve to increase both the temperature and humidity of the inhaled air to minimise the differential in these parameters with those of the surface of the lungs. Normally some heat and moisture is also captured from exhaled air prior to its release to the atmosphere. The mucous lining of the nasal passages also serves to remove particulate matter, such as fine dust particles, pollutants and microorganisms, from the inhaled air, and the action of cilia transports mucous and any particles away from the lungs.

When a person has received a laryngectomy, in effect all inhaled air enters the lungs via the tracheostoma, and the nose is effectively not involved in the inhalation process. Exhaled air may pass through the tracheostoma or, if a voice prosthesis has been fitted, the stoma can be occluded so that the exhaled air is diverted through the voice prosthesis into the pharynx and the mouth, enabling the person to speak. It is desirable that the flow of the exhaled air be controlled by means of a tracheostoma valve. In these situations, the valve can be arranged to remain open during breathing but can be closed to divert the airflow, through a small additional increase in exhaled air flow.

In this respect tracheostoma devices, such as filter devices, HME, breathing protectors, and speech valves, have been developed to enable moisturizing of inhaled air, removal of small particles and bacteriological substances in said inhaled air, and providing the person with the ability to speak by closing the air passage through the tracheostoma by manual operation.

These tracheostoma devices are held in place by a tracheostoma device holder, arranged above the tracheostoma of the person. The tracheostoma device holder is normally attached to the skin of the person by a plaster, having an adhesive surface on the side of the plaster intended to be directed towards the person using it. Either, the tracheostoma device holder is welded to the plaster, or the tracheostoma device holder is arranged on an adhesive surface on the side of the plaster intended to be directed outwards from the person using it. On the skin adhesive surface a covering sheet may be applied, which is removed just before application of the tracheostoma device holder. The covering sheet facilitates transportation, and maintains skin adhesive ability of the skin adhesive surface.

There is however a problem associated with the application of the tracheostoma device holder after the removal of the covering sheet, since the neck of the person receiving the tracheostoma device holder by no means is planar. It is difficult to adhere the tracheostoma device holder in the pit in between the sternocleidomastoid muscles, at persons with sunken stomas, i.e. stomas that somewhat has sunken into the neck of the person, since the adhesive surface of the tracheostoma device holder inevitably will adhere to the walls of the pit before reaching the bottom of the pit with the central portion of the system. Sunken stomas are very frequent in the group of persons not having the two vertical sternocleidomastoid muscles on the neck cut during laryngectomy.

Furthermore, a bad fit between the non-planar surface of the neck and the adhesive surface of the tracheostoma device holder may lead to leakages between the neck and said adhesive surface, thereby causing a loss of speaking pressure.

To solve this problem, tracheostoma device holders may utilize plates with a conical shape for adhering the tracheostoma device to the neck in order for it to follow the shape of the sunken stoma more closely.

However, this introduces a number of challenges. For example, the plate may not simply be stamped out from a sheet due to the three-dimensional structure required. Instead, the conical plate has to be provided by other more complex manufacturing methods. Alternatively, the conical shape of the adhesive surface has previously been achieved by means of providing a soft plaster with a rigid core structure for achieving and maintaining the conical shape of the soft plate.

The introduction of the rigid core structure negatively affects the formability of the plate since a rigid material has to be introduced. This may cause discomfort for the patient due to chafing against the neck.

Hence, an improved tracheostoma device holder would be advantageous, and in particular a tracheostoma device holder allowing for convenient application of the tracheostoma device holder with improved positioning ability, while simultaneously decreasing the risk of loosening of skin adhesion close to the tracheostoma, also in persons with sunken tracheostomas, thus keeping speech pressure at a convenient level, as well as making up for irregularities in skin shape adjacent the stoma, while simultaneously compensating for patient specific anatomy and/or local and momentary skin wounds or irritations.

Further, it would also be advantageous with a tracheostoma device holder which can be manufactured in a less complex and more cost-efficient manner while still providing a comfortable fit to the stoma of a patient.

The following documents disclose tracheostoma device holders known in the art WO 2017/135867, EP 1 513 575, JP 2013 052033, EP 3 061 484, US 2015/306327, US 5 819 734, DE 20 2004 007566, US 2008/072911, US 2012/103341 and US 2005/277901.

### Summary of the Invention

Accordingly, the present invention preferably seeks to mitigate, alleviate or eliminate one or more of the above-identified deficiencies and disadvantages, singly or in any combination, and solves at least the above mentioned problems by providing a tracheostoma device holder according to claim 1 for holding a tracheostoma device superimposed of a tracheostoma of a person, the tracheostoma device holder comprising a plate for attachment over a tracheostoma via proximal side thereof, said plate being provided with a through hole; a tracheostoma device fitting arranged superimposed the through hole, said tracheostoma device fitting extending distally from a distal side of the plate; whereby the tracheostoma device holder is formed by a co-molding process, and wherein a thickness of the plate decreases in the central to lateral direction.

Thereby, a tracheostoma device holder which can be optimized in terms of materials and shapes can be achieved. By allowing for a wider range of available materials and shapes of the components, the tracheostoma device holder may become suitable for a wider range of stoma shapes as well as able to follow the shape of stoma of a patient more closely.

A method for co-molding such a tracheostoma holding device is also provided at least for the same reasons.

Advantageous features of the invention are defined in the dependent claims.

### Brief Description of the Drawings

These and other aspects, features and advantages of which the invention is capable of will be apparent and elucidated from the following description of embodiments of the present invention, reference being made to the accompanying drawings, in which
Fig. 1 is a cross-section view of a tracheostoma device holder according to an embodiment of the present invention;
Fig. 2 is a cross-section view of a tracheostoma device holder according to an embodiment of the present invention;
Fig. 3 is a cross-section view of a tracheostoma device holder according to an embodiment of the present invention; and
Fig. 4 is a cross-section view of a tracheostoma device holder according to an embodiment of the present invention.

### Description of the Embodiments

The following description focuses on an embodiment of the present invention applicable to a tracheostoma device holder, for holding a tracheostoma device, such as a tracheostoma valve, over the stoma of a person. A tracheostoma device may in this context be a HME, speech valve, etc.

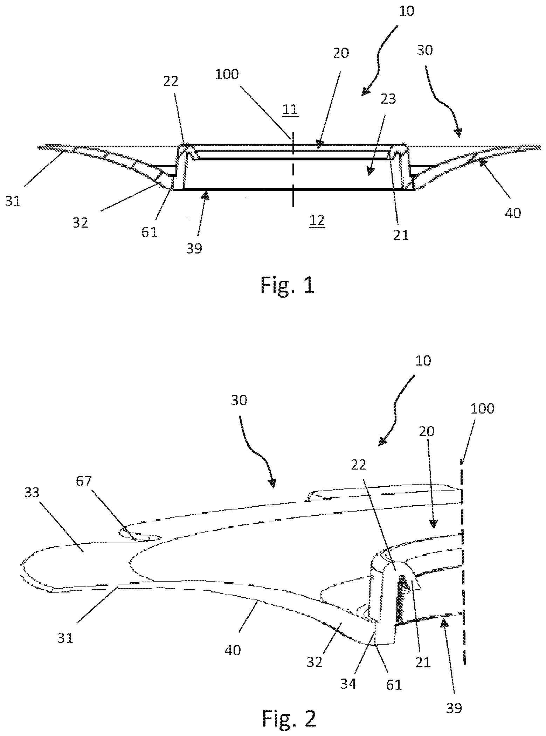

Fig. 1 discloses a cross-section view of a tracheostoma device holder 10 for holding a tracheostoma device superimposed of a tracheostoma of a person, said tracheostoma device holder 10 comprising a plate 30 for attachment over the tracheostoma via a proximal side thereof, said plate 30 being provided with a through hole 39.

The tracheostoma device holder 10 further comprises a tracheostoma device fitting 20 extending distally from a distal side of the plate 30. The tracheostoma device fitting is preferably a tubular tracheostoma device fitting 20. The tracheostoma device fitting 20 comprises a through passage 23, extending through the tracheostoma device holder 10. The through passage 23 of the tracheostoma device fitting 20 is superimposed the through hole 39 of the plate 30, so as to form a distal opening 11 communicating with a proximal opening 12 in the tracheostoma device holder 10. Preferably, the aperture formed by the tubular tracheostoma device fitting 20 and said through hole 39 being provided in the plate 30 coincide about an axis 100 of the through hole 39.

The tracheostoma device holder 10 thus comprises a proximal end and a distal end. In this context proximal refers to a position or direction towards the stoma, i.e. towards the user of the tracheostoma device holder 10, whilst distal refers to a position or direction away from the stoma, i.e. away from the user of the tracheostoma device holder 10. Lateral refers to a position or direction radially away from the axis 100, whilst central refers to a position or direction towards the central axis 100. The axis 100 extends in a distal-proximal direction through the tracheostoma device holder 10. With advantage said axis 100 may coincide with a central axis of said tracheostoma device holder 10.

The tubular tracheostoma device fitting 20 is disposed circumferentially of the through hole 39, in the form of for example a cylindrical sleeve. Thus, the tracheostoma device fitting 20 extends distally from the plate 30 circumferentially of said through hole 39. The plate 30 will extend mainly laterally as a flange from the tubular tracheostoma device fitting 20, in relation to the axis 100 of the through hole 39. Similarly, the tubular tracheostoma device fitting 20 extends axially and distally from the plate 30, in accordance with above.

Referring again to Fig. 1, the plate 30 may be of a substantially circular shape, with the through hole 39 extending through the center of said plate.

The tracheostoma device holder 10 is formed by a co-molding process, wherein the plate 30 is molded onto the tubular tracheostoma device fitting 20. The plate 30 and the fitting 20 may be made of different materials. With advantage, the plate 30 is in a flexible material while the tubular tracheostoma device fitting 20 is in a more rigid material.

Thus, the plate 30 may be made of a flexible and elastic material, such as rubber, silicone or a thermoplastic material, such as a thermoplastic elastomer (TPE), while the fitting 20 may be made of a more rigid material, such as PE, PP, PS; or TPE.

The fitting 20 is preferably in a more rigid material due to its required retaining properties for holding the tracheostoma device in place. Thus, the more rigid material allows for a press-fit or snap-fit between the fitting 20 and the inserted tracheostoma device provided by the elastic deformation of the more rigid material of the fitting 20.

The two materials may have different melting points. Accordingly, since the plate 30 is molded onto the fitting 20, the elastic material forming the plate 30 may have a lower melting point than the more rigid material forming the fitting 20. Thus, no complex cooling arrangement has to be used in order to achieve the co-molding without affecting the properties of the fitting 20. By having two different materials with similar melting points the fitting may be formed in a first injection molding step. Thereafter the fitting is placed into a second mold, where after the plate is molded onto the fitting. In this way, the similar melting points are used to re-melt the surface layer of the fitting to fuse or bond the fitting and the plate together.

The tracheostoma device holder 10 may thus be co-molded by means of a first molding step where the tubular tracheostoma device fitting 20 is injection molded in a first tool. Said first step is then followed by a second molding step where said fitting 20 is moved to a second tool, wherein the material for forming the plate 30 is added. Thus, plate 30 is molded to the fitting 20 without any requirements on any additional fastening means.

Compared to a conventional tracheostoma device holder, the co-molded tracheostoma device holder 10 allows for a significant increase in freedom in terms of design as well as materials of the tracheostoma device holder 10. Accordingly, the co-molding of the plate 30 and the fitting 20 allows for optimizing of the tracheostoma device holder 10 so as to achieve the desired properties.

In line with the above, the molding of the plate 30 allows for forming of the plate 30 in shapes more closely following the shape of the stoma of a patient. The shape of the molding tool forming the plate 30 may allow for variation of material thickness and shape in three-dimensions in a way which is impossible with a plate stamped out of a sheet. Further, the molding of said plate 30 allows for a larger variation of materials being available for forming the plate 30, since the materials do not have to be chosen to enable being forming by means of conventional sheet-forming processes. Thus, a material which is significantly more flexible can be chosen for the plate which allows for the plate to more closely follow the shape of the stoma. Notably, the material may be so flexible that this can be achieved independently of the shape of the stoma of the patient, whereby a tracheostoma device holder which is suitable for a wide range of shapes of stoma is achieved. The flexible material also reduces the risk for leakage and/or perceived discomfort of the patient due to the co-molding allowing for a softer material which closely follows the stoma.

The co-molding further allows for an interface 34 to be placed adjacent or at least very close to the distally extending tracheostoma device fitting 20, i.e. close to the stoma, when the device holder is worn. This allows for a more comfortable fit of the device holder 10 also in the area closest to the stoma.

Also, the co-molding allows for the fitting 20 and the plate 30 to be joined in a manner without requiring any additional welding or joining operations. Such welding or joining operations, for example ultrasonic welding, may be facilitated by having the plate 30 in a rigid material, considerably reducing the comfort of the patient as well as the plate's ability to adapt to varying shapes of stomas. Hence, the co-molding allows for a device holder 10 where the material of the plate 30 does not have to be chosen so as to allow for joining operations, whereby a more flexible and adaptable device holder 10 is enabled. Additionally, the co-molding allows for a higher freedom in placement and configuration of the interface between the two parts, since welding requires at least one of the materials to be thin enough in the direction orthogonal to the interface for being welded onto the other material whilst this restriction on placement and configuration does not apply for co-molding. The latter also results in the possibility to increase the plate surface towards the fitting or decrease the lateral extension of the plate, since there is no need for sheet-overlap between the fitting and the plate, whereby the effective surface area of the plate, and the flexibility and compliance towards the skin of the user is increased. This allows for the possibility to decrease skin adhesive property of the skin adhesive layer on the proximal side of the plate, leading to the possibility to decrease skin irritation and facilitated removal of the device holder.

Similarly, the co-molding process also allows for the more rigid material to be relatively soft and comfortable for the patient to wear. The co-molding does not require any welding whereby a wider range of materials may be chosen for the fitting, accordingly it allows for materials which fulfill the rigidity requirements but are softer than materials required in a conventional fitting to be used.

The proximal side of the plate 30 may be provided with a sheet 40 being adhesive on both sides, and at least skin adhesive on one of these. Such a sheet 40 may be dimensioned in accordance with the proximal side of the plate 30, such that the interaction surface between the plate 30 and the skin may be high. Also, in contrast to welding, there is no risk of affecting the skin adhesive property close to the weld. When welding plastic sheets to each other, the welding namely "kills" the adhesive property in the vicinity, such as below the weld.

Again referring to Fig. 1, the plate 30 may have a substantially conical shape that is inclined proximally towards the axis 100. In other words, the radius from the central axis to the plate 30 in a plane transversal to the axis 100 is decreasing in the distal to proximal direction, whereby said radius at the distal end is greater than the radius at the proximal end. Hence, the plate 30 may extend laterally and in a distal direction at an angle from the tubular tracheostoma device fitting 20. Said angle may preferably be from 30 to 80 degrees, such as 50 to 70 degrees, between the lateral side of the device fitting 20 and the plate 30.

The conical shape of the plate 30 resembles the shape of the stoma of a patient, whereby said plate is able to more closely follow the shape of the stoma making it more comfortable to wear and less likely to leak.

To achieve an even closer and more comfortable fit to the stoma of a patient, the plate has a rounded conical shape, preferably in the form of a trumpet-shape. This means that the angle between the lateral side of the device fitting 20 and the plate 30 increases moving laterally. This even more resembles the shape of the stoma, furthermore it reduces the risk for discomfort due to sharp distal edges of the plate 30 digging into the sternocleidomastoid muscles since the folding around said sternocleidomastoid muscles is ensured due to the rounded convex shaped proximal surface reaching around the same.

Notably, the rounded convex shape of the plate 30 is not achievable with a conventional plaster with a rigid core or a conventional plate. However due to the injection molding of the plate 30 this may be achieved simply by the provision of a tool with the desired shape.

This rounded convex shape is shown in Fig. 1, where the plate 30 extends laterally at an angle in a laterally extending arc-shape from the tubular tracheostoma device fitting 20 in a distal direction.

Preferably, the plate 30 may have a hardness below 40 Shore so as to allow for the sufficient flexibility. However, a too soft material will be harder to apply to a stoma. Furthermore it may also wrinkle during application and wearing. To achieve the sufficient flexibility of the material while reducing the risk for wrinkling and a more difficult application of the device holder 10, the plate 30 may preferably have a hardness from 10 Shore 00 to 50 Shore A, such as 50 Shore 00 to 30 Shore A. The fitting 20 may preferably have a hardness from 55 to 90 Shore A, such as from 70 to 90 Shore A.

Yet again referring to Fig. 1, the tubular tracheostoma device fitting 20 may comprise a retaining lip 21 extending circumferentially from a distal zone of said fitting 20. The retaining lip 21 comprises a bent portion 22 extending proximally and laterally towards the axis 100 of the through hole of the plate. Said retaining lip 21 being adapted to receive and retain the tracheostoma device.

The receiving and the retaining of the tracheostoma device is provided by the elastic deformation of the more rigid material of the fitting 20 and the bent portion 22 of the retaining lip 21. When the tracheostoma device is inserted into the fitting 20 in a distal to proximal direction along the axis 100 of the through hole 39 of the plate 30, the bent portion 22 will deform laterally towards the inner portion of the tubular tracheostoma device fitting. This leaves room for the tracheostoma device to pass through the retaining lip 21 and be fully inserted into the tubular tracheostoma device fitting 20.

The bent portion 22 of the retaining lip 21 then retains the tracheostoma device inside the fitting 20. Due to the inwardly bent structure any distal movement of the tracheostoma device past the retaining lip will be hindered. This being due to any load on the retaining lip 21 caused by said movement will simply lead to the retaining lip 21 deforming by means of elastically bending the retaining lip 21 so as to urge the retaining lip 21 to exert a proximally directed normal force holding the tracheostoma device in place.

The retaining lip 21 thus allows for a tracheostoma device holder 10 mitigating the risk for a tracheostoma device to accidentally fall out of the tubular tracheostoma device fitting 20, when the tracheostoma device holder 10 is used by a patient.

For the fitting to provide a retaining lip with the desired retaining properties, the fitting must be in a more rigid material. However, in order to provide such desired retaining properties while still providing a tracheostoma device holder which is comfortable to wear for the patient, the plate must be in a different and more flexible material. According to aspects of the invention, a more flexible and comfortable material, which still holds the desired retaining properties, may be used without negatively effecting the adhesion effect between plate and fitting.

Accordingly, the co-molding of a tracheostoma device holder with a retaining lip solves the problem of achieving a tracheostoma device holder which mitigates the risk for the tracheostoma device to accidentally fall out of the tubular tracheostoma device fitting during wearing without reducing the perceived comfort of the patient or introducing additional adhering or joining steps during manufacturing.

Turning to Fig. 2 a more detailed cross-section of the tracheostoma device holder 10 is shown. Referring to said figure, the plate 30 comprises a tip portion 31 forming the lateral, e.g. circumferential, end of the plate 30. Hence, said tip portion 31 of the plate 30 is annularly shaped and extending circumferentially around a base portion 32 of the plate adjacent to the tubular tracheostoma device fitting 20. Said base portion 32 thus has an annular shape extending laterally around the tubular tracheostoma device fitting 20.

The tip portion 31 further comprises a plurality of flaps 33 extending laterally from a lateral outer portion of the plate 30. Since the plate may have a substantially convex shape said flaps 33 may extend laterally in an angle from the axis 100 in a distal direction, preferably along the lateral direction of the remaining tip portion 31.

The flaps 33 are preferably separated by a plurality of cut-ins 67 extending centrally towards the axis 100 from a lateral circumferential edge of the tip portion 31. Said flaps 33 further increase the adaptability of the plate 30 since each flap 33 may be adhered to a surface without affecting the orientation of the other flaps of the tip portion 31.

In a conventional plate the (partial) folding of one section of the tip portion may cause a strain propagating through the material of the plate which may force other sections of said plate to release from their intended position on the stoma of the patient. Said flaps 33 thus provides additional means for the plate 30 to more closely follow the shape of the stoma of a patient, furthermore they provide an improvement in the adhering properties of the plate 30.

Compared to a plate which has been stamped out from a sheet, the molded plate with flaps may be provided directly in the injection molding operation

Hence, the flaps 33 provide a mean to increase the ability for the plate to follow the shape of the stoma of a patient more closely without increasing the complexity of the manufacturing process.

Again referring to Fig. 2, the thickness of the plate 30 varies in a lateral direction, i.e. a direction substantially orthogonal to the axis 100 moving radially outwards. The base portion 32 is preferably thicker than the tip portion 31. This is particularly advantageous since the thicker material of the base portion 32 allows for a stronger bond between the fitting 20 and the plate 30 due to the relatively large contact area between said fitting 20 and plate 30, while the thinner material at the tip portion 31 makes the remaining portion of the plate 30 more flexible and formable. In addition the thicker material of the base portion 32 makes the holder 10 less susceptible for differing tolerances during the molding process due to the thicker dimension of the base portion allowing for sufficient adhering between plate and fitting even when a deficit of material has occurred during molding. Additionally, a thicker base allows for an increased force distribution around the fitting 20, upon exposure to axial or transversal forces during use.

The thickness of the plate 30 may thus vary across said plate from approximately 0,1 mm to 3 mm, more preferably approximately from 0,2 mm to 2 mm and even more preferably from approximately 0,3 mm to 1,5 mm.

Mentioned dimensions allows for sufficient adhering surfaces around the fitting while providing a thin enough tip portion for the sufficient flexibility required to closely follow the stoma of the patient. However, the tip portion is not so thin that it becomes difficult to apply or may wrinkle when the tracheostoma device holder 10 is worn by a patient.

Notably, the thickness of the plate 30 may vary gradually from the base portion 32 to the tip portion 31. The gradual thinning of the material mitigates any risk for cracks or weakening of the material due to notches in the plate 30 formed by edges as a result of abrupt transitions between portions of different thickness.

Further, the gradual thinning of the material mitigates the risk for warping of the material of the plate. Warping is always a risk during injection molding of any component with non-uniform thickness, however the risk for warping to occur is severely reduced if the thickness reduces or increases gradually instead of instantly by means of steep transitions between thick portions and thin portions.

Thus, the thickness may vary gradually from the base portion 32 towards the tip portion 31 from 0.8-3 mm to 0.1-0.8 mm, such as from 1-2 mm to 0.2-0.4 mm.

As further depicted in Fig. 2, an interface 34 between the plate 30 and the tubular tracheostoma device fitting 20 may be formed by a proximodistal face 61 of the plate 30 and the tubular tracheostoma device fitting 20. The inner/central face 61 extends along the axis 100, i.e. in a proximodistal direction and abuts to the lateral face of the tubular tracheostoma device fitting 20 also extending along said axis 100, i.e. in a proximodistal direction. The interface 34 is thus a proximodistal interface 34 formed by a substantially annularly shaped boundary surrounding the tubular tracheostoma device fitting 20, extending substantially orthogonal to the skin of the user.

Due to the tracheostoma device holder 10 being susceptible to forces parallel to the axis 100, which may be transferred into a shear force in the interface between the fitting 20 and the plate 30, a large proximodistal interface may absorb such forces in the best way.

However, with conventional adhering operations such a proximodistal interface is difficult to achieve. Instead, the fitting is usually adhered on top of the distal surface of the plate. In such a solution little resistance is provided to shearing forces between the proximal surface of the fitting and the distal surface of the plate and the adhesion is prone to peel off. Such shearing load may induce cracks between said components which causes leakage. Thus, the orientation of the interface allows for an interface between plate and fitting which maintains structural integrity even during relatively large proximodistal loads, as well as shearing forces.

Further, the proximodistal interface 34 is especially advantageous from an injection molding perspective. The proximodistal interlace 34 does not force any flow of material in a proximodistal direction around the proximal end of the tubular tracheostoma device fitting 20 during injection molding. Instead, the end of plate has a planar portion, i.e. a uniform portion which decreases the risk for warping of the plate 30 close to its proximodistal face 61.

Also, the proximodistal boundary /interface 34 mitigates the impact of tolerance deviations in the injection molding process, i.e. co-molding process, since the risk for the material flowing proximally of the proximal surface of the tubular tracheostoma device fitting 20, so as to intersect the aperture formed by the tubular tracheostoma device fitting 20, may be mitigated. This is due to the tool not having to allow fluid access proximally of said proximal surface of the tubular tracheostoma device fitting 20.

Hence, the proximodistal interface further achieves a tracheostoma device holder 10 less susceptible to tolerance issues and potential flow deviations during injection molding.

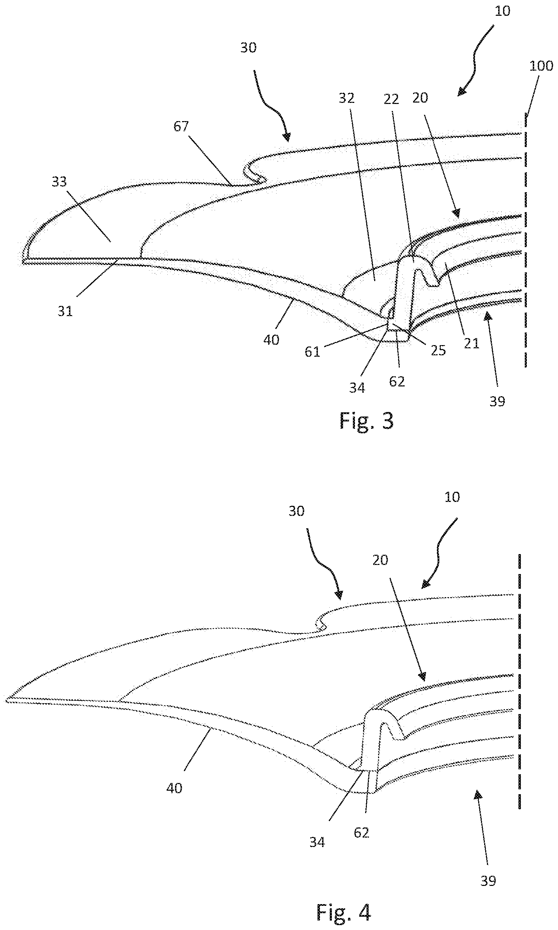

Turning to Fig. 3, the interface 34 between the plate 30 and the tubular tracheostoma device fitting 20 may be substantially L-shaped. Accordingly, the interface 34 comprises a proximodistal face 61, i.e. a face extending in a proximal and distal direction, and a laterocentral face 62, i.e. a face extending in a lateral and central direction, continuing from said proximodistal face 61. The proximodistal face 61 forms a surface in the proximodistal direction, whereby the inner zone section 62 extends laterally from said proximodistal face 61, preferably along a proximal portion adjacent to said proximodistal face 61 centrally towards said axis 100.

As shown in said Fig. 3, a thickness of the plate 30 in a proximodistal direction proximally of the laterocentral face (62) is smaller than the proximodistal extension of the proximodistal face (61). This allows for a higher flexibility and skin adhesion closer to the stoma while simultaneously increasing the effective surface area of the proximodistal face 61, allowing for effective contact between a lateral side of said tubular tracheostoma device fitting 20 and the plate 30. The laterocentral face 62 may further extend so as to be substantially flush with the border of the through passage 23 of the tubular tracheostoma device fitting 20.

The L-shaped interface between the device fitting 20 and the plate 30 increases the interface 34, i.e. the contact area, between fitting 20 and plate 30 further, which strengthens the adhering between said components and further mitigates the risk for leakage or damage due to load during wearing of the tracheostoma device holder 10.

Notably, the L-shaped interface also provides support in two-dimensions significantly increasing the torsional rigidity of the device holder in general and the adhering between the plate 30 and the fitting 20 in particular. The torsional rigidity may be especially important during the fitting of the tracheostoma device in the tracheostoma device fitting 20, during this process the adhering interface may be subjected to rotational load and/or diagonal loads. Hence, the risk for inducing cracks during fitting of the tracheostoma device into the tracheostoma device fitting is reduced by means of said L-shaped interface.

To co-mold the plate to the fitting with such an interface poses a significant production advantage. If the plate and the fitting are co-molded, the interface may simply be achieved by the tool directly during the molding. If a similar interface was to be achieved without co-molding it would require both an additional machining operation where an indentation has to be formed around the central opening of the plate as well as an adhering operation where the plate and fitting are joined together. Such operations make the production of the tracheostoma device holder more expensive and more complex. Thus, the co-molding allows for a more cost-efficient and less complex manufacturing method to achieve such an interface.

The co-molded L-shaped interface is also advantageous in relation to a conventional molding process where the plate and fitting are molded separately and then adhered together. The structure of the inner portion of the plate required to achieve the L-shaped interface brings about a relatively sharp edge between the molding tool and the plate during injection molding, whereby several negative structural effects may occur during molding such as warping or cracks. Thus, the co-molding of the L-shaped interface allows for a molding method where said interface is achieved with a lower risk for warping, crack inducing or other negative structural effects during molding.

To further increase the contact surface between the tubular tracheostoma device fitting 20 and the plate 30, a proximal portion of the tubular tracheostoma device fitting 20 may be provided with a flange 25 at its proximal end. The flange 25 protrudes laterally and outwardly, i.e. in a substantially orthogonally direction away from the axis 100, from the remaining outer portion of the tubular tracheostoma device fitting.

Accordingly, said proximal portion extends along a distal portion of the laterocentral face 62, whereby said proximal portion also has a lateral portion being in direct contact with the proximodistal face 61.

As shown in Fig. 4, the interface 34 between the plate 30 and the tubular tracheostoma device fitting 20 may as well be arranged completely laterocentral. Accordingly, the interface 34 does not comprise a proximodistal face 61, but only a laterocentral face 62. The laterocentral face 62 extends laterally from the border of the through hole 39, along the proximal side of the fitting 20.

Injection molding of relatively large surfaces is associated with a risk for warping of especially the middle of the surface. To mitigate this risk the tubular tracheostoma device fitting 20 may comprise at least one gusset. Each gusset may extend laterally from the tubular tracheostoma device fitting to contact the plate 30.

Said gussets also allow for a more simple application of the plate 30 to the stoma of the patient, due to the gusset(s) fixating the shape of the elastic material forming the plate 30 during application.

Due to the plate being co-molded onto the tubular tracheostoma device fitting 20, said at least one gusset may be adapted to extend inside the plate 30. Thereby, the tracheostoma device holder 10 may be provided with gussets extending from the lateral surface of the fitting 20 into the plate 30. Thus, the aforementioned benefits of the gussets may be achieved without negatively impacting the comfort of the patient due to the gussets coming into contact with the stoma of the patient. Furthermore, the adhesive properties remain the same due to the proximal surface of the plate 30 extending proximally of the gusset(s).

According to one aspect of the invention, a method for co-molding a tracheostoma device holder for holding a tracheostoma device superimposed of a tracheostoma of a person according to any of the aforementioned embodiments is provided. The tracheostoma device holder 10 comprises a plate 30 for attachment over a tracheostoma via proximal side thereof, said plate 30 being provided with a through hole 39 and a tubular tracheostoma device fitting 20 arranged circumferentially of the through hole 39, said tubular tracheostoma device fitting 20 extending distally from a distal side of the plate 30. The plate 30 may be in a flexible material and the tracheostoma device fitting 20 being in a more rigid material. The method comprises injection molding the tubular tracheostoma device fitting 20 in a first injection molding tool with a first molding cavity. Thereafter the tracheostoma device fitting 20 may be transferred into a second injection molding tool with a second molding cavity, where after the plate 30 may be injection molded onto said tracheostoma tubular tracheostoma device fitting 20, thereby forming the tracheostoma device holder 10. It is also possible to injection mold the fitting 20 and the plate 30 in the same tool, wherein the mold cavity is changed and increased after the injection molding of the fitting 20, by withdrawal of mold inserts, where after the plate 30 is injection molded onto the fitting 20.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A tracheostoma device holder (10) for holding a tracheostoma device superimposed of a tracheostoma of a person, said tracheostoma device holder (10) comprising:
a plate (30) for attachment over a tracheostoma via proximal side thereof, said plate (30) being provided with a through hole (39);
a tracheostoma device fitting (20) being provided with a through passage (23) arranged superimposed the through hole (39), said tracheostoma device fitting (20) extending distally from the plate (30); whereby
the tracheostoma device holder (10) is formed by a co-molding process, wherein the plate (30) is molded onto the tracheostoma device fitting (20), and **characterized in that** the thickness of the plate decreases in the central to lateral direction.

2. The tracheostoma device holder (10) according to claim 1, wherein the plate (30) is in a more flexible material than the tracheostoma device fitting (20).

3. The tracheostoma device holder (10) according to claim 1 or 2, wherein the plate (30) comprises a thermoplastic elastomer or silicone, and the tracheostoma device fitting (20) comprises another thermoplastic material.

4. The tracheostoma device holder (10) according to any of the preceding claims, comprising an interface (34) between the plate (30) and the tracheostoma device fitting (20), wherein a material of the plate (30) at said interface has a melting point different from a melting point of a material of the tracheostoma device fitting (20) at said interface (34).

5. The tracheostoma device holder (10) according to any of the preceding claims, wherein the proximal side of the plate (30) is provided with a sheet (40) being adhesive on both sides, and skin adhesive on its proximal side.

6. The tracheostoma device holder (10) according to any of the preceding claims, wherein the plate (30) has a conical shape.

7. The tracheostoma device holder (10) according to any of the preceding claims, wherein the plate (30) has a hardness from 10 Shore 00 to 50 Shore A, and the fitting (20) has a hardness from 55 to 90 Shore A.

8. The tracheostoma device holder (10) according to any of the preceding claims, wherein a thickness of the plate (30) is from 0.1 mm to 3 mm.

9. The tracheostoma device holder (10) according to claim 8, wherein thickness varies gradually from a base portion (32) of the plate (30) towards a tip portion (31) of the plate (30) from 0.8-3 mm to 0.1-0.8 mm.

10. The tracheostoma device holder (10) according to any of the preceding claims, wherein an interface (34) between the plate (30) and the tracheostoma device fitting (20) comprises a proximodistal face (61) of the plate (30) and abuts to a lateral face of the tracheostoma device fitting (20).

11. The tracheostoma device holder (10) according to claim 10, wherein the interface (34) comprises a proximodistal face (61) and a laterocentral face (62).

12. The tracheostoma device holder (10) according to claim 11, wherein a thickness of the plate proximally of the laterocentral face (62) is smaller than the proximodistal extension of the proximodistal face (61).

13. A method for manufacturing a tracheostoma device holder (10) according to any one of claims 1 to 12, for holding a tracheostoma device superimposed of a tracheostoma of a person, said method comprising:
injection molding the tracheostoma device fitting (20) in a first step; and
injection molding the plate (30) onto said tracheostoma device fitting (20) in a second step.

## Patentansprüche

1. Tracheostomavorrichtungshalter (10) zum Halten einer Tracheostomavorrichtung über einem Tracheostoma einer Person, wobei der Tracheostomavorrichtungshalter (10) Folgendes umfasst:
eine Platte (30) zum Anbringen über einem Tracheostoma über die proximale Seite davon, wobei die Platte (30) mit einem Durchgangsloch (39) versehen ist,
ein Tracheostomavorrichtungsanschlussstück (20), das mit einem Durchgang (23) versehen ist, der über dem Durchgangsloch (39) angeordnet ist, wobei sich das Tracheostomavorrichtungsanschlussstück (20) distal von der Platte (30) erstreckt, wobei
der Tracheostomavorrichtungshalter (10) durch ein Co-Molding-Verfahren ausgebildet wird, wobei die Platte (30) auf das Tracheostomavorrichtungsanschlussstück (20) aufgeformt wird, und **dadurch gekennzeichnet, dass** die Dicke der Platte in der mittleren zur seitlichen Richtung abnimmt.

2. Tracheostomavorrichtungshalter (10) nach Anspruch 1, wobei die Platte (30) aus einem flexibleren Material als das Tracheostomavorrichtungsanschlussstück (20) ist.

3. Tracheostomavorrichtungshalter (10) nach Anspruch 1 oder 2, wobei die Platte (30) ein thermoplastisches Elastomer oder Silikon umfasst und das Tracheostomavorrichtungsanschlussstück (20) ein anderes thermoplastisches Material umfasst.

4. Tracheostomavorrichtungshalter (10) nach einem der vorhergehenden Ansprüche, umfassend eine Grenzfläche (34) zwischen der Platte (30) und dem Tracheostomavorrichtungsanschlussstück (20), wobei der Schmelzpunkt eines Materials der Platte (30) an der Grenzfläche von einem Schmelzpunkt eines Materials des Tracheostomavorrichtungsanschlussstücks (20) an der Grenzfläche (34) verschieden ist.

5. Tracheostomavorrichtungshalter (10) nach einem der vorhergehenden Ansprüche, wobei die proximale Seite der Platte (30) mit einer Folie (40) versehen ist, die an beiden Seiten klebend ist und an ihrer proximalen Seite an der Haut klebend ist.

6. Tracheostomavorrichtungshalter (10) nach einem der vorhergehenden Ansprüche, wobei die Platte (30) eine konische Form hat.

7. Tracheostomavorrichtungshalter (10) nach einem der vorhergehenden Ansprüche, wobei die Platte (30) eine Härte von 10 Shore 00 bis 50 Shore A hat und das Anschlussstück (20) eine Härte von 55 bis 90 Shore A hat.

8. Tracheostomavorrichtungshalter (10) nach einem der vorhergehenden Ansprüche, wobei eine Dicke der Platte (30) von 0,1 mm bis 3 mm beträgt.

9. Tracheostomavorrichtungshalter (10) nach Anspruch 8, wobei die Dicke allmählich von einem Basisabschnitt (32) der Platte (30) zu einem Spitzenabschnitt (31) der Platte (30) von 0,8 - 3 mm bis 0,1 - 0,8 mm variiert.

10. Tracheostomavorrichtungshalter (10) nach einem der vorhergehenden Ansprüche, wobei eine Grenzfläche (34) zwischen der Platte (30) und dem Tracheostomavorrichtungsanschlussstück (20) eine proximodistale Fläche (61) der Platte (30) umfasst und an einer lateralen Fläche des Tracheostomavorrichtungsanschlussstücks (20) anliegt.

11. Tracheostomavorrichtungshalter (10) nach Anspruch 10, wobei die Grenzfläche (34) eine proximodistale Fläche (61) und eine laterozentrale Fläche (62) umfasst.

12. Tracheostomavorrichtungshalter (10) nach Anspruch 11, wobei eine Dicke der Platte proximal der laterozentralen Fläche (62) kleiner als die proximodistale Erstreckung der proximodistalen Fläche (61) ist.

13. Verfahren zur Herstellung eines Tracheostomavorrichtungshalters (10) nach einem der Ansprüche 1 bis 12 zum Halten einer Tracheostomavorrichtung über einem Tracheostoma einer Person, wobei das Verfahren Folgendes umfasst:
Spritzgießen des Tracheostomavorrichtungsanschlussstücks (20) in einem ersten Schritt und
Spritzgießen der Platte (30) auf das Tracheostomavorrichtungsanschlussstück (20) in einem zweiten Schritt.

## Revendications

1. Support de dispositif de trachéostomie (10) permettant de tenir un dispositif de trachéostomie superposé à une trachéostomie d'une personne, ledit support de dispositif de trachéostomie (10) comprenant :
une plaque (30) destinée à être fixée sur une trachéostomie par le biais de son côté proximal, ladite plaque (30) étant pourvue d'un trou traversant (39) ;
un raccord (20) de dispositif de trachéostomie pourvu d'un passage traversant (23) agencé superposé au trou traversant (39), ledit raccord (20) de dispositif de trachéostomie s'étendant distalement à partir de la plaque (30) ; moyennant quoi
le support de dispositif de trachéostomie (10) est formé par un processus de co-moulage, dans lequel la plaque (30) est moulée sur le raccord (20) de dispositif de trachéostomie, et **caractérisé en ce que** l'épaisseur de la plaque diminue dans la direction du centre vers le côté.

2. Support de dispositif de trachéostomie (10) selon la revendication 1, dans lequel la plaque (30) est dans un matériau plus souple que le raccord de dispositif de trachéostomie (20).

3. Support de dispositif de trachéostomie (10) selon la revendication 1 ou 2, dans lequel la plaque (30) comprend un élastomère thermoplastique ou de la silicone, et le raccord (20) de dispositif de trachéostomie comprend un autre matériau thermoplastique.

4. Support de dispositif de trachéostomie (10) selon l'une quelconque des revendications précédentes, comprenant une interface (34) entre la plaque (30) et le raccord (20) de dispositif de trachéostomie, dans lequel un matériau de la plaque (30) au niveau de ladite interface a un point de fusion différent d'un point de fusion d'un matériau du raccord (20) de dispositif de trachéostomie au niveau de ladite interface (34).

5. Support de dispositif de trachéostomie (10) selon l'une quelconque des revendications précédentes, dans lequel le côté proximal de la plaque (30) est pourvu d'une feuille (40) étant adhésive des deux côtés, et adhésive pour la peau sur son côté proximal.

6. Support de dispositif de trachéostomie (10) selon l'une quelconque des revendications précédentes, dans lequel la plaque (30) a une forme conique.

7. Support de dispositif de trachéostomie (10) selon l'une quelconque des revendications précédentes, dans lequel la plaque (30) a une dureté de 10 Shore 00 à 50 Shore A, et le raccord (20) a une dureté de 55 à 90 Shore A.

8. Support de dispositif de trachéostomie (10) selon l'une quelconque des revendications précédentes, dans lequel une épaisseur de la plaque (30) est de 0,1 mm à 3 mm.

9. Support de dispositif de trachéostomie (10) selon la revendication 8, dans lequel l'épaisseur varie progressivement d'une partie de base (32) de la plaque (30) vers une partie de pointe (31) de la plaque (30) de 0,8 à 3 mm à 0,1 jusqu'à 0,8 mm.

10. Support de dispositif de trachéostomie (10) selon l'une quelconque des revendications précédentes, dans lequel une interface (34) entre la plaque (30) et le raccord (20) de dispositif de trachéostomie comprend une face proximodistale (61) de la plaque et vient en butée contre une face latérale du raccord (20) de dispositif de trachéostomie.

11. Support de dispositif de trachéostomie (10) selon la revendication 10, dans lequel l'interface (34) comprend une face proximodistale (61) et une face latérocentrale (62).

12. Support de dispositif de trachéostomie (10) selon la revendication 11, dans lequel une épaisseur de la plaque proximalement à la face latérocentrale (62) est inférieure à l'extension proximodistale de la face proximodistale (61).

13. Procédé de fabrication d'un support de dispositif de trachéostomie (10) selon l'une quelconque des revendications 1 à 12, permettant le maintien d'un dispositif de trachéostomie en superposition à une trachéostomie d'une personne, ledit procédé comprenant :
le moulage par injection du raccord (20) de dispositif de trachéostomie dans une première étape ; et
le moulage par injection de la plaque (30) sur ledit raccord (20) de dispositif de trachéostomie dans une seconde étape.
